# EUROPEAN PATENT APPLICATION

(11) **EP 4 553 851 A1**
(43) Date of publication of application: **14.05.2025**
(21) Application number: 23208791.6
(22) Date of filing: 09.11.2023
(51) Int. Cl.: G16H 50/70

(54) **BIOPHYSICAL MODELS**

(71) Applicant: Koninklijke Philips N.V., 5656 AG Eindhoven (NL)
(72) Inventor: FRANITZA, Katharina, Eindhoven (NL); SCHUMMERS, Georg, Eindhoven (NL)
(74) Representative: Philips Intellectual Property & Standards

(57) **Abstract**

A method by which a first parameter of a first model of a statistical form model and a physiological simulation model of a cardiovascular system, is determined based on the other, second model of the statistical form model and the physiological simulation model. The first model may then be modified based on the determined first parameter to determine a modified first model.

## Description

### FIELD OF THE INVENTION

This invention relates to the field of biophysical models, and in particular to models of a cardiovascular system.

### BACKGROUND OF THE INVENTION

Models of cardiovascular systems may be used by researchers and medical professionals to aid with the diagnosis and treatment of patients. Models of heart systems may be used as teaching aids to educate physicians about different possible disease patterns in such systems and their effect on heart anatomy. Further, when modelling techniques are used to produce patient specific digital twins, the resulting models may be used for better visualisation and analysis of a patient's current disease state and possible progression. These accurate models based on patient data may then help in planning interventions or other treatments, for example heart valve repairs or pharmacological treatments. Two example classes of model that may be applied to cardiovascular systems are statistical form models and physiological simulation models.

Statistical form models are based on large datasets of anatomical data. The data is summarised into a mean form model that may have different variations. The statistical form model may then be fitted to data of the specific cardiovascular system being modelled to identify geometric features of target objects of the system. Since they are based on averages, statistical form models often lack the flexibility to deal with real patient data in marginal disease cases.

Physiological simulation models start with an initial set of parameters that describe the system to be modelled and then use known biological and physical concepts to predict how the system will evolve over time. Modelling the evolution of the system may use information describing biological characteristics of the system being modelled. For example, the model may use the known effects of a certain disorder to predict how a specific system with a known disorder may develop. The model may be constrained via a fitting process involving real patient data. The initialisation of a physiological simulation model is often difficult, as many of the parameters involved in such a model, for example those describing material behaviour, can only be measured invasively or not at all. Further, since the modelling itself is a simplification of real-world processes, not every parameter of the model has a real-world equivalent.

The potential value of both statistical form models and physiological simulation models depends on how accurately they can be fitted to patient specific data and therefore how reliable they are in reproducing characteristics of a real-life system.

### SUMMARY OF THE INVENTION

The invention is defined by the claims.

According to examples in accordance with an aspect of the invention, there is provided a method for modifying a model of a cardiovascular system.

The method for modifying a model of a cardiovascular system comprises: for a first model of a statistical form model of the cardiovascular system and a physiological simulation model of the cardiovascular system, determining a first parameter for the first model based on the other, second model of the statistical form model and the physiological simulation model; and modifying the first model based on the determined first parameter to determine a modified first model.

Proposed concepts thus aim to provide schemes, solutions, concepts, designs, methods and systems pertaining to a method for modifying a model of a cardiovascular system.

In particular, embodiments aim to provide a method by which a first parameter of a first model of a statistical form model and a physiological simulation model of a cardiovascular system, is determined based on the other, second model of the statistical form model and the physiological simulation model. The first model may then be modified based on the determined first parameter to determine a modified first model.

It was realised that some of the weaknesses of statistical form models of cardiovascular systems may be overcome when their fitting is guided by information contained within a physiological simulation model. Similarly, some of the difficulties faced when attempting to form and propagate a physiological simulation model of a cardiovascular system, may be overcome when a statistical form model of the same system is used to aid the formation and evolution of the model. The proposed invention therefore leverages both a statistical form model and a physiological simulation model of a system to produce a modified model of the system that may not suffer some of the drawbacks of the original models in isolation.

Statistical form models and physiological simulation models of cardiovascular systems each have shortcomings when it comes to providing a complete and accurate model of a given cardiovascular system. Statistical form models may not be able to accurately map patient data in the case of extreme disease cases and physiological simulation models are often hard to initialise and constrain. The present invention proposes combining the two modelling approaches by determining parameters for one of these two models based on the other of the two models i.e., determining a parameter for the physiological simulation model based on the statistical form model, or determining a parameter for the statistical form model based on the physiological simulation model. The resulting modified model may not suffer from some of the issues faced by the original models taken in isolation and therefore may provide a more accurate representation of the target system which may be used in treatment and diagnostic procedures.

The modified model may consider both the physical relationships between components of the system, as traditionally contained within the physiological simulation model, and the anatomical and statistical knowledge of the statistical form model.

In summary, by determining at least one parameter for a first of the statistical form model and the physiological simulation model, based on the other of the statistical form model and the physiological simulation model, there is provided an improved method for modifying models of cardiovascular systems that overcomes the deficiencies of using either of the two model types individually.

Ultimately, an improved method for modifying a model of a cardiovascular system may be supported by the proposed concept(s).

In some embodiments, the method further comprises: determining a second parameter of the second model based on at least one of the first model and the modified first model; and modifying the second model based on the determined second parameter to determine a modified second model. In this way, the method may result in generation of two modified models, a modified statistical form model and a modified physiological simulation model, which may both be a more accurate than the original models.

In some embodiments, a parameter of the statistical form model may comprise a disorder-specific fitting parameter. In this way, the physiological simulation model may provide a disorder-specific information about the cardiovascular system which may be used to modify the fitting of the statistical form model, resulting in a more accurate statistical form model. Traditionally, due to their basis on a mean form model, statistical form models struggle to accurately model some diseased cardiovascular systems which may have abnormal characteristics.

In some embodiments, a parameter of the physiological simulation model may comprise at least one of: an initial set-up parameter; a constraint parameter; and a relationship between a plurality of constraint parameters. In this way, the statistical form model may provide initialisation parameters for the physiological simulation model which is traditionally a difficult process. Further, the statistical form model may be used to place constraints on the evolution of the physiological simulation model, stabilising the evolution of the model and ensuring the resulting predictions are biologically plausible.

In some embodiments, an initial set-up parameter and a constraint parameter may describe a geometric parameter of the system, and preferably wherein the geometric parameter of the system comprises at least one of: a length; an area; a volume; an orientation; a shape; a displacement vector field; a moving pattern; a strain; and a stress. Thus, geometric data obtained from the statistical form model may be used to obtain parameters for the physiological simulation model.

In some embodiments, a parameter of the physiological simulation model relates to at least one of; a ventricular chamber; an atrial chamber; a valve; an electrical stimulus; a pericardium; and a hemodynamic value. In this way, the parameters of the physiological simulation model may describe various anatomical features of the cardiovascular system.

In some embodiments, the statistical form model may be based on acquired data associated with the cardiovascular system. In this way, a mean form statistical model may be fitted to system specific data of the cardiovascular system being modelled to form the statistical form model of the cardiovascular system.

In some embodiments, the acquired data may comprise at least one of: three-dimensional echocardiogram data; four-dimensional echocardiogram data; computed tomography data; position emission tomography data; magnetic resonance imaging data; and single photon emission computed tomography data. In this way, system specific data may be acquired via any of a number of different medical imaging techniques.

In some embodiments, the physiological simulation model may comprise information describing at least one disorder-specific characteristic of the cardiovascular system. In this way, the physiological simulation model may be disorder dependent and may utilise known information on how certain disorders are expected to impact the evolution of the cardiovascular system.

In some embodiments, the physiological simulation model may be generated according to a method comprising: generating a plurality of physiological simulation models; selecting based on the statistical form model, a system-specific physiological simulation model from the plurality of physiological simulation models; and modifying the system-specific physiological simulation model based on a disorder specific characteristic to determine the physiological simulation model. In this way, there is provided a method by which the initial physiological simulation model for the system may be generated such that it contains information about a known disorder of the system.

In some embodiments, the method may further comprise determining a third parameter for the first model based on the second model and modifying the first model based on the determined third parameter to determine a third model. In this way the method of modifying the two models may be used iteratively to determine further parameters for each of the two models and in this way further refine them.

In another embodiment, a non-transitory computer readable medium is provided storing instructions for performing a method for modifying a model of a cardiovascular system. When executed by at least one processor, the instructions cause the at least one processor to: for a first model of a statistical form model of the cardiovascular system and a physiological simulation model of the cardiovascular system, determine a first parameter for the first model based on the other second model of the statistical form model and the physiological simulation model; and modify the first model based on the determined first parameter to determine a modified first model.

According to another aspect, there is provided a system for modifying a model of a cardiovascular system, wherein the system comprises a processor configured to: for a first model of a statistical form model of the cardiovascular system and a physiological simulation model of the cardiovascular system, determine a first parameter for the first model based on the other, second model of the statistical form model and the physiological simulation model; and modify the first model based on the determined first parameter to determine a modified first model.

Thus, there may be proposed concepts for providing a method of modifying a model of a cardiovascular system comprising modifying a first model of a statistical form model and a physiological simulation model based on a first parameter which is determined based on the other, second model of the statistical form model and the physiological simulation model.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for modifying a model of a cardiovascular system;
Fig. 2A and Fig. 2B are illustrations of the segmentation process applied to acquired data of a cardiovascular system involved in the formation of a statistical form model;
Fig. 3 is a simplified flow diagram of an alternative method for modifying a model of a cardiovascular system;
Fig. 4 is a simplified flow diagram of an alternative method for modifying a model of a cardiovascular system; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to a method for modifying a model of a cardiovascular system. According to proposed concepts, a number of possible solutions may be implemented separately or jointly. That is, although these possible solutions may be described below separately, two or more of these possible solutions may be implemented in one combination or another.

Embodiments of the invention aim to provide a method for modifying a model of a cardiovascular system, wherein the method involves determining at least one parameter for a first model of the system based on a second model of the system and wherein the first and second models are one or the other of a statistical form model and a physiological simulation model. The method described herein results in the formation of a modified model which may overcome certain disadvantages of the originally formed statistical form model and physiological simulation model.

Proposed concepts thus aim to provide a method of mutual modification of a statistical form model and a physiological simulation model of a cardiovascular system, thereby producing modified models that lack some of the pitfalls of each of the two originally formed models.

A statistical form model is trained using a large database of geometric data from which the mean forms of different characteristic shapes associated with the system are extracted. The level of variation of these characteristic shapes is also analysed. The mean form and variation data may then be used in fitting a statistical form model to system specific data. The segmentation process involved in this fitting results in the identification of various characteristic shapes in the image that each correspond to various anatomic features of the system. The size, shape, and orientation of each anatomical feature is also determined in the segmentation process. In the case of a cardiovascular system, the statistical form model may provide estimates of the volume and length scales associated with the various anatomical features of the cardiovascular system. For example, in applying a statistical form model to an echocardiogram image of a heart, the atrial and ventricular regions may be identified, and their size, shape, and orientation determined. This process may be repeated for many, temporally separated, images of a system to build up a full digital model of the various geometric characteristics of the anatomical features of the system and how they evolve.

Statistical form models involve the analysis of statistically likely geometrical configurations, and therefore this type of model struggles to accurately reproduce abnormal systems, even when the fitting process is configured to consider the variation of shapes present in the training dataset. Further, fitting a statistical form model to system specific data may involve applying the model to various temporally separated images of the system. The statistical form model may develop the fitting of each image independently of its temporal neighbours. This, in conjunction with poor time resolution of the underlying data, may result in the fitted model having inconsistent movement patterns and only poorly reproducing the dynamical features of the system. In the case of a statistical form model applied to a cardiovascular system, events that happen on timescales shorter that the temporal resolution of the imaging technique being used to acquire the system-specific data, will be lost and not represented in the statistical form model of the system. These events may include circulatory events such as valve closure events. Further, if the data to which the model is fitted has low temporal resolution, the statistical form model will not contain details of the actual movement patterns of the features of the system. Some of these detailed movement patterns, such as septal flashes or the character of chamber relaxation patterns, are crucial in diagnostic settings and hence as it stands these models may be of limited use in this context.

Physiological simulation models use knowledge of the biophysical behaviours of the components of the system being modelled to estimate how the system will evolve over time. For a cardiovascular system, this may involve biological information on the various phases of the heart cycle, and physical information on the forces experienced by each component of the cardiovascular system and the expected behaviour of each component when subjected to those forces. A physiological simulation model may consider disorder- specific factors which determine how the various parameters of a system may vary depending on the type and stage of disease associated with the system. The physiological simulation model may be further optimised for a specific system using acquired data of the system.

The most challenging aspect of all simulation models and especially those representing biological processes, is model initialization. Many parameters of the simulation model, such as those describing material behaviour, can only be measured invasively or not at all. In addition, modelling itself is a simplification of real-world processes and as such not every modelling parameter has a real-world equivalent. Even given accurate initialisation of the model, the process of fitting the model to system specific data may often benefit from stabilising constraints to ensure the resulting model is physiologically plausible.

The present invention provides a mechanism by which the information of these two models may be combined to overcome some of the issues outlined above.

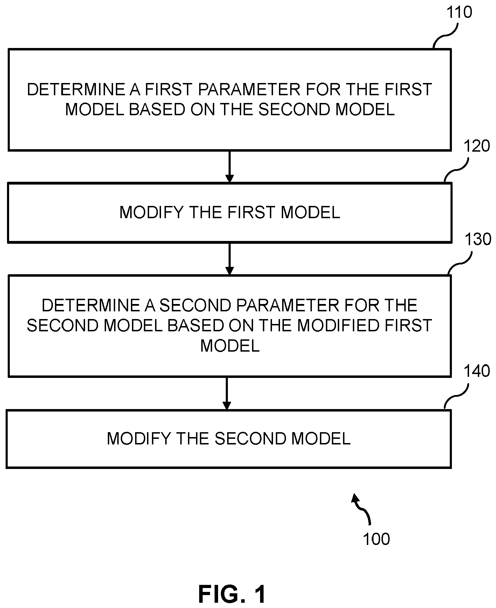

Referring now to Fig. 1, there is provided a simplified flow diagram of a method 100 for modifying a model of a cardiovascular system according to a proposed embodiment.

The method 100 starts with a step 110 comprising, for a first model of a statistical form model of the cardiovascular system and a physiological simulation model of the cardiovascular system, determining a first parameter for the first model based on the other, second model of the statistical form model and the physiological simulation model.

In this exemplary embodiment, the first model is the physiological model, and the second model is the statistical form model. The first parameter determined for the physiological model in this exemplary embodiment comprises an initial set-up parameter describing a geometric parameter of the system. The geometric parameter comprises at least one of: a length; an area; a volume; an orientation; a shape; a displacement vector field; a moving pattern; a strain; and a stress, relating to at least one of; a ventricular chamber; an atrial chamber; a valve; an electrical stimulus; a pericardium; and a hemodynamic value. Therefore, in this exemplary embodiment a statistical form model of the cardiovascular system is used to generate at least one geometric parameter describing the cardiovascular system, which acts as an initial set-up parameter for the physiological model.

In detail, a statistical form model is fitted to three-dimensional echocardiogram data of the cardiovascular system. The statistical form model identifies geometric shapes in the echocardiogram data and using statistical information assigns these shapes to anatomical features of the system. The size and shape of these identified features are then used as an initialisation point for a physiological model of the system.

Once the first parameter has been determined, the method proceeds to a step 120 comprising modifying the first model based on the determined first parameter to determine a modified first model. Thus, a modified physiological simulation model is produced that is initialised based on information extracted from the statistical form model. The physiological simulation model relies on known biological and physical relationships and processes to describe the evolution of different parameters of the system from the initialisation point defined by the first parameter.

In this exemplary embodiment, the method then proceeds to a step 130 comprising determining a second parameter for the second model based on the modified first model. In the subsequent step 140, the second model is modified based on the determined second parameter to determine a modified second model.

In this exemplary embodiment, the second model is the statistical form model and the second parameter for the second model comprises a disorder-specific fitting parameter. In this way, the physiological simulation model which is based on biophysical relations that describe disorder specific characteristics of the system, may be used to shift the working range of the statistical form model in the direction of a specific disorder that is known to be present in the system. Thus, the method described herein provides a mechanism by which the fit of a statistical form model of a cardiovascular system may be directed to a disorder-specific fitting when the system being modelled is known to have an associated disorder.

In the method outlined above, the first model is the physiological simulation model and the second model is the statistical form model. In alternative embodiments the first model may be the statistical form model and the second model may be the physiological simulation model.

Similarly, other steps of the method detailed above may be implemented differently in alternative embodiments. For instance, steps 130 and 140 need not be included and the method may simply involve basing parameters of one of model of the cardiovascular system on information generated by another model of the cardiovascular system, where the two distinct models involved are a statistical form model and a physiological simulation model. In embodiments for which the step 130 is included, it may alternatively comprise determining a second parameter for the second model based on the first model i.e., the second parameter may be determined based on either the original unmodified first model or the modified first model.

Further, in alternative embodiments the acquired data of the cardiovascular system to which the statistical form model is fitted may be data acquired by any one of a number of well-known medical imaging techniques. For example, in alternative embodiments the acquired data may comprise at least one of: four-dimensional echocardiogram data; computed tomography data; position emission tomography data; magnetic resonance imaging data; and single photon emission computed tomography.

The determined parameter of the physiological simulation model is not limited to an initialisation parameter but may comprise a constraint parameter or a relationship between a plurality of constraint parameters. In this way, the parameter of the physiological simulation model determined based on the statistical form model, may provide biologically plausible limits to the physiological model to enable further stabilisation of the evolution of the system predicted by the model. For example, the statistical form model may provide geometric maximum chamber dimensions for the heart being modelled, or a constraining relationship between the volumes of the two ventricular chambers of the heart. This information can be obtained from the statistical form model due to its basis on a large database of geometric data. This may result in a simplification of the optimisation process of the simulation model towards a system-specific simulation as it enables anatomically improbable configurations to be ruled out.

The determined parameter for the statistical form model is not limited to a disorder-specific fitting parameter but may comprise a parameter used to improve the dynamic element of the statistical form model. For example, the physiological simulation model may simulate how the volume of a cardiac component evolves over time. The simulated volume curve of the physiological simulation model may then be used to aid interpolation between distinct phases of the heart cycle which are segmented in the statistical form model. This interpolation can smooth the movement patterns of the statistical form model. In addition, the consistency of the statistical simulation model may be checked to prevent artifacts in the acquired data from causing incorrect segmentation.

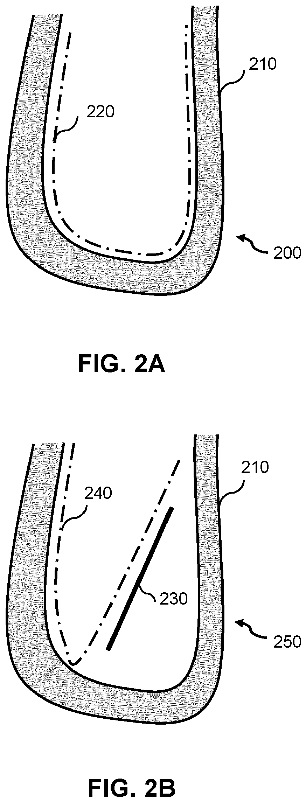

Referring now to Fig. 2A and Fig. 2B are illustrations of the segmentation process applied to acquired data of a cardiovascular system involved in the formation of a statistical form model according to a proposed embodiment.

Fig. 2A illustrates an image 200 of an anatomical volume 210 in a patient cardiovascular system, taken at an initial time *t₁*. A statistical form model is used to segment the image 200 and correctly identify a geometric volume 220. The statistical form model may calculate various geometrical parameters e.g. shape, lengths, and volume of the geometrical volume 220.

Fig. 2B illustrates an image 250 of the same anatomical volume 210, taken at a time *t₂*, which is later than the time *t₁*. In this image an artifact 230 is present. The presence of the artifact 230 results in the statistical form model incorrectly segmenting the image 250 to identify an incorrect geometric volume 240. Thus, a statistical form model that is derived per frame of acquired imaging data without knowledge of the images taken in the time frames either side, may produce incorrect segmentation of the image 250. A resulting dynamic model combining the segmentations of image 200 and the image 250 may therefore comprise inconsistent movement patterns.

Using the method described herein, the statistical form model of the patient cardiovascular system may be modified. The modification applied to the model is determined based on information obtained from a physiological simulation model of the system. The physiological model considers known biophysical processes to model the evolution of the system between the times *t₁* and *t₂.* By using the physiological simulation model to direct the fitting of the statistical form model, the incorrect segmentation of Fig. 2A may be avoided, even with the presence of artifacts in the imaging data.

Thus, by using a physiological simulation model to dynamically stabilise the segmentation of the statistical form model, more consistent modelling results may be obtained. This may achieve the result of reducing the effect of artifacts on the resulting segmentation.

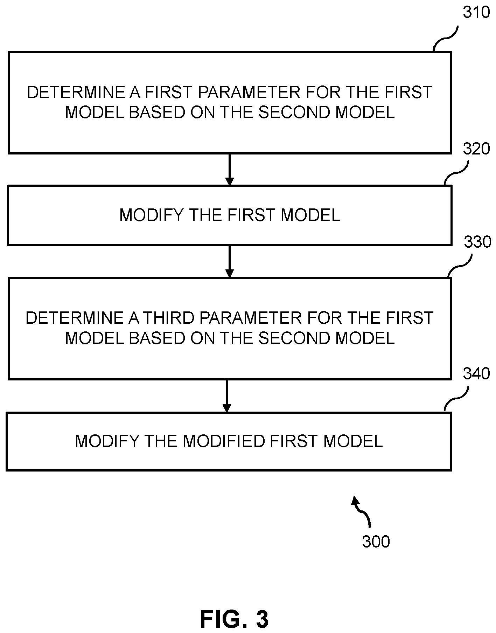

Fig. 3 is a simplified flow diagram of an alternative method 300 for modifying a model of a cardiovascular system according to a proposed embodiment.

The method 300 commences with steps 310 and 320 which are equivalent to the steps 110 and 120 of the method 100. Step 310 comprises for a first model of a statistical form model of the cardiovascular system and a physiological simulation model of the cardiovascular system, determining a first parameter for the first model based on the other, second model of the statistical form model and the physiological simulation model. Step 320 comprises modifying the first model based on the determined first parameter to determine a modified first model.

The method 300 then proceeds to a step 330 comprising determining a third parameter for the first model based on the second model. In step 340 this determined third parameter is used to modify the modified first model to determine a third model. Although in this exemplary embodiment only two parameters are determined, it can be envisioned that a similar method may be used to generate any number of parameters to progressively modify a model of the cardiovascular system. In this way, the method of the present invention may be used iteratively to progressively improve the fit of the first model. This may involve generating multiple different parameters for a model of the first model type, which each refer to different components of the system to progressively improve the accuracy of the model with each modification.

Alternatively, the iterative approach may be used to determine parameters for the first model that correspond to the values of a given parameter of the system at different points in time. For example, in a certain embodiment the statistical form model may be used to provide multiple time-dependent constraint parameters relating to a chamber volume of the cardiovascular system.

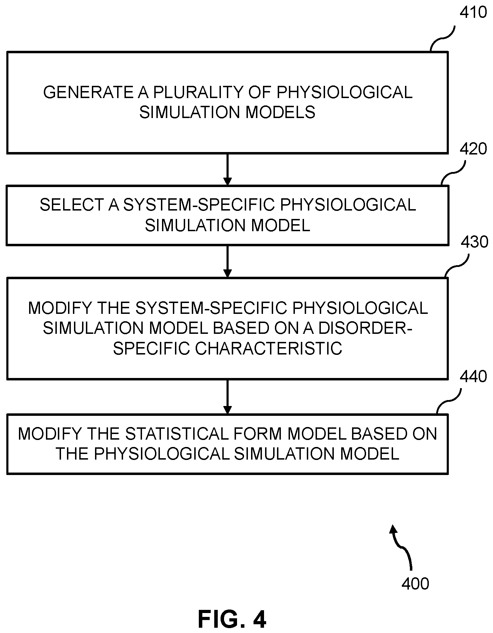

Referring now to Fig. 4, there is depicted an alternative method 400 of modifying a model of a cardiovascular system according to a proposed embodiment.

The method starts with a step 410 which comprises generating a plurality of physiological simulation models for the cardiovascular system. Each of the plurality of physiological simulation models may correspond to a different set of model parameters which may for example relate to hearts of different sizes or configurations.

The method then proceeds to a step 420 comprising selecting, based on a statistical form model of the cardiovascular system, a system specific physiological simulation model from the plurality of physiological simulation models. This may involve using the statistical form model to determine a set of parameters for the system-specific simulation model and then selecting the model from the plurality of generated physiological simulation models that corresponds to set of model parameters that most closely aligns with the set of parameters determined from the statistical form model.

Step 430 comprises modifying the system-specific physiological simulation model based on a disorder-specific characteristic to determine the physiological simulation model. In this way the generated physiological simulation model may be based on known disorder-specific information describing the various parameters of the system and how they might evolve.

Once the disorder-specific modification has been made to the system-specific physiological simulation model, the method proceeds to a step 440 comprising of modifying the statistical form model based on the determined physiological simulation model. In this way, a disorder-specific fitting parameter may be determined and applied to the statistical form model to optimise the fitting of the statistical form model to acquired data of the cardiovascular system being modelled.

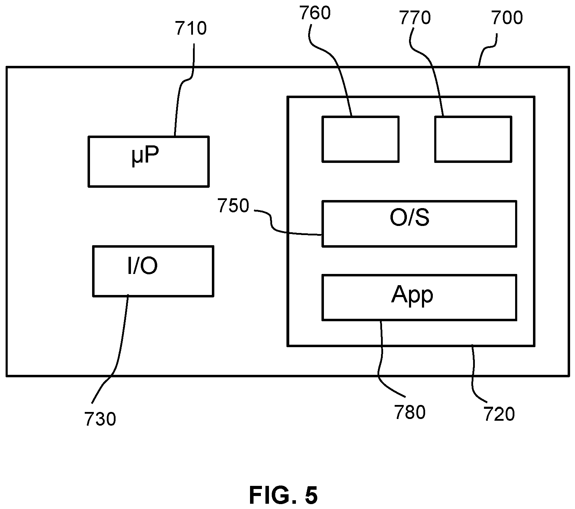

Referring now to Fig. 5, there is illustrated an example of a computer within which one or more parts of an embodiment may be employed.

Fig. 5 illustrates an example of a computer 700 within which one or more parts of an embodiment may be employed. Various operations discussed above may utilize the capabilities of the computer 700. In this regard, it is to be understood that system functional blocks can run on a single computer or may be distributed over several computers and locations (e.g. connected via internet).

The computer 700 includes, but is not limited to, PCs, workstations, laptops, PDAs, palm devices, servers, storages, and the like. Generally, in terms of hardware architecture, the computer 700 may include one or more processors 710, memory 720 and one or more I/O devices 730 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 710 is a hardware device for executing software that can be stored in the memory 720. The processor 710 can be virtually any custom made or commercially available processor, a central processing unit (CPU), a digital signal processor (DSP), or an auxiliary processor among several processors associated with the computer 700, and the processor 710 may be a semiconductor based microprocessor (in the form of a microchip) or a microprocessor.

The memory 720 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 720 may incorporate electronic, magnetic, optical, and/or other types of storage media. Note that the memory 720 can have a distributed architecture, where various components are situated remote from one another, but can be accessed by the processor 710.

The software in the memory 720 may include one or more separate programs, each of which comprises an ordered listing of executable instructions for implementing logical functions. The software in the memory 720 includes a suitable operating system (O/S) 750, compiler 760, source code 770, and one or more applications 780 in accordance with exemplary embodiments. As illustrated, the application 780 comprises numerous functional components for implementing the features and operations of the exemplary embodiments. The application 780 of the computer 700 may represent various applications, computational units, logic, functional units, processes, operations, virtual entities, and/or modules in accordance with exemplary embodiments, but the application 780 is not meant to be a limitation.

The operating system 750 controls the execution of other computer programs, and provides scheduling, input-output control, file and data management, memory management, and communication control and related services. It is contemplated by the inventors that the application 780 for implementing exemplary embodiments may be applicable on all commercially available operating systems.

Application 780 may be a source program, executable program (object code), script, or any other entity comprising a set of instructions to be performed. When a source program, then the program is usually translated via a compiler (such as the compiler 760), assembler, interpreter, or the like, which may or may not be included within the memory 720, so as to operate properly in connection with the O/S 750. Furthermore, the application 780 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 730 may include input devices such as, for example but not limited to, a mouse, keyboard, scanner, microphone, camera, etc. Furthermore, the I/O devices 730 may also include output devices, for example but not limited to a printer, display, etc. Finally, the I/O devices 730 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 730 also include components for communicating over various networks, such as the Internet or intranet.

If the computer 700 is a PC, workstation, intelligent device or the like, the software in the memory 720 may further include a basic input output system (BIOS) (omitted for simplicity). The BIOS is a set of essential software routines that initialize and test hardware at start-up, start the O/S 750, and support the transfer of data among the hardware devices. The BIOS is stored in some type of read-only-memory, such as ROM, PROM, EPROM, EEPROM or the like, so that the BIOS can be executed when the computer 700 is activated.

When the computer 700 is in operation, the processor 710 is configured to execute software stored within the memory 720, to communicate data to and from the memory 720, and to generally control operations of the computer 700 pursuant to the software. The application 780 and the O/S 750 are read, in whole or in part, by the processor 710, perhaps buffered within the processor 710, and then executed.

When the application 780 is implemented in software it should be noted that the application 780 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 780 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The method of Figs. 1,3, and 4 may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A method for modifying a model of a cardiovascular system, wherein the method comprises:
for a first model of a statistical form model of the cardiovascular system and a physiological simulation model of the cardiovascular system, determining (110) a first parameter for the first model based on the other, second model of the statistical form model and the physiological simulation model; and
modifying (120) the first model based on the determined first parameter to determine a modified first model.

2. The method of claim 1, wherein the method further comprises:
determining (130) a second parameter for the second model based on at least one of the first model and the modified first model; and
modifying (140) the second model based on the determined second parameter to determine a modified second model.

3. The method of claim 1, wherein a parameter of the statistical form model comprises a disorder-specific fitting parameter.

4. The method of claim 1, wherein a parameter of the physiological simulation model comprises at least one of: an initial set-up parameter; a constraint parameter; and a relationship between a plurality of constraint parameters.

5. The method of claim 4, wherein the initial set-up parameter and constraint parameter describe a geometric parameter of the system, and preferably wherein the geometric parameter of the system comprises at least one of: a length; an area; a volume; an orientation; a shape; a displacement vector field; a moving pattern; a strain; and a stress.

6. The method of any of claims 1 to 5, wherein a parameter of the physiological simulation model relates to at least one of: a ventricular chamber; an atrial chamber; a valve; an electrical stimulus; a pericardium; and a hemodynamic value.

7. The method of any of claims 1 to 6, wherein the statistical form model is based on acquired data associated with the cardiovascular system.

8. The method of claim 7, wherein the acquired data comprises at least one of: three-dimensional echocardiogram data; four-dimensional echocardiogram data; computed tomography data; position emission tomography data; magnetic resonance imaging data; and single photon emission computed tomography data.

9. The method of any of claims 1 to 8, wherein the physiological simulation model comprises information describing at least one disorder-specific characteristic of the cardiovascular system.

10. The method of claim 9, wherein the physiological simulation model is generated according to a method comprising:
generating (410) a plurality of physiological simulation models;
selecting (420), based on the statistical form model, a system-specific physiological simulation model from the plurality of physiological simulation models; and
modifying (430) the system-specific physiological simulation model based on a disorder-specific characteristic to determine the physiological simulation model.

11. The method of any claims 1-10, wherein the method further comprises:
determining (330) a third parameter for the first model based on the second model; and
modifying (340) the modified first model based on the determined third parameter to determine a third model.

12. A computer program comprising computer program code means adapted, when said computer program is run on a computer, to implement the method of any of claims 1-11.

13. A system for modifying a model of a cardiovascular system, wherein the system comprises:
a processor (710) configured to:
for a first model of a statistical form model of the cardiovascular system and a physiological simulation model of the cardiovascular system, determine a first parameter for the first model based on the other, second model of the statistical form model and the physiological simulation model; and
modify the first model based on the determined first parameter to determine a modified first model.

14. The system of claim 13, wherein the processor is further configured to:
determine a second parameter for the second model based on at least one of the first model and the modified first model; and
modify the second model based on the determined second parameter to determine a modified second model.

15. The system of claim 15, wherein a parameter of the statistical form model comprises a disorder-specific fitting parameter.
